(19) Europäisches Patentamt
European Patent Office
Office européen des brevets

(11) Numéro de publication: **0 267 098 B1**

(12) **FASCICULE DE BREVET EUROPEEN**

(45) Date de publication de fascicule du brevet: **22.01.92**

(51) Int. Cl.[5]: **C07D 209/42**, C07C 69/75, C07C 235/40, A61K 31/40

(21) Numéro de dépôt: **87402354.2**

(22) Date de dépôt: **21.10.87**

(54) **Procédé de préparation de dérivés de l'octahydroindole et intermédiaires de préparation.**

(30) Priorité: **22.10.86 FR 8614650**

(43) Date de publication de la demande: **11.05.88 Bulletin 88/19**

(45) Mention de la délivrance du brevet: **22.01.92 Bulletin 92/04**

(84) Etats contractants désignés: **AT BE CH DE ES GB GR IT LI LU NL SE**

(56) Documents cités:
**EP-A- 0 037 231**
**EP-A- 0 084 164**
**US-A- 4 490 386**

**JOURNAL OF MEDICINAL CHEMISTRY, vol. 28, no. 11, novembre 1985, pages 1606-1611, American Chemical Society; G.M. KSANDER et al.: "Angiotensin converting enzyme inhibitors: N-substituted D-glutamic acid gamma dipeptides"**

(73) Titulaire: **ROUSSEL-UCLAF**
**35, Boulevard des Invalides**
**F-75007 Paris(FR)**

(72) Inventeur: **Brion, Francis**
**10bis, rue d'Avron**
**F-93220 Gagny(FR)**
Inventeur: **Buendia, Jean**
**3bis, Impasse Emilie**
**F-94170 Le Perreux Sur Marne(FR)**
Inventeur: **Marie, Christian**
**39-41, rue des Guillaumes**
**F-93120 Noisy-Le-Sec(FR)**

(74) Mandataire: **Vieillefosse, Jean-Claude**
**Département des Brevets ROUSSEL UCLAF**
**B.P no 9**
**F-93230 Romainville(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

**TETRAHEDRON LETTERS**, vol. 24, no. 48, 1983, pages 5339-5342, Pergamon Press Ltd.; R. HENNING et al.: "Diastereoselective synthesis of bicyclic amino acids via ring contraction of alpha-chlorolactams"

**TETRAHEDRON LETTERS**, vol. 24, no. 48, 1983, pages 5343-5346, Pergamon Press Ltd.; R. HENNING et al.: "Coupling of beta-acetamido radicals with alpha-chloro acrylonitrile - a new access to disubstituted proline derivatives"

## Description

La présente demande concerne un procédé de préparation de dérivés de l'octahydroindole et des intermédiaires de préparation.

La demande de brevet européen n¤ 0 084 164 a déjà décrit la préparation de dérivés de formule :

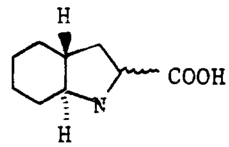

et leur application à la préparation de produits doués de propriétés inhibitrices de l'enzyme de conversion de l'angiotensine. Une telle préparation a également été décrite dans Tetrahedron Letters 24, 5343 et 24 5347 (1983) et peut être schématisée comme suit :

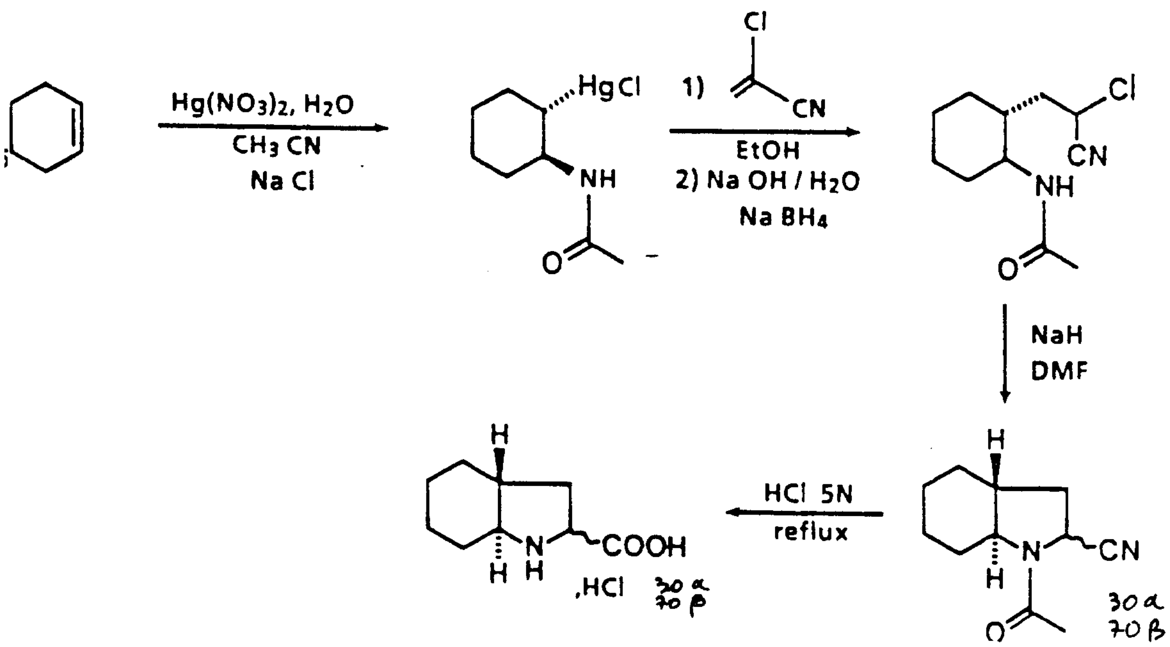


Cette synthèse a cependant l'inconvénient au niveau industriel d'utiliser du mercure sous forme de nitrate en quantité stoéchiométrique dès la première étape. De plus, à la dernière étape le nitrile est présent à 30% dans le mélange d'épimères et la même proportion est retrouvée, au niveau de l'acide.

Dans ce qui précède, le trait ondulé signifie que le nitrile existe en mélange alpha - béta, et le trait plein au niveau du carbone 3a signifie que l'hydrogène est en alpha ou en béta.

C'est pourquoi la présente demande a pour objet un procédé de préparation asymétrique des dérivés de formule (I) :

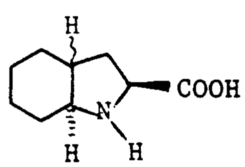

(I)

dans laquelle les hydrogènes en 3a et 7a sont de configuration cis ou trans, ainsi que de leurs sels d'addition avec les acides, caractérisé en en que l'on soumet un produit de formule (II) :

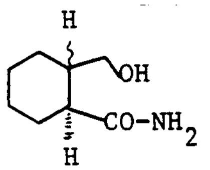

(II)

à une réaction d'Hofmann, puis à l'action de formaldéhyde en présence d'ions cyanures et d'un halogénure d'acyle aliphatique renfermant de 1 à 5 atomes de carbone ou de benzoyle, pour obtenir un dérivé de formule (III) :

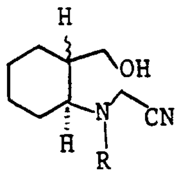

(III)

dans laquelle R représente un radical acyle aliphatique renfermant de 1 à 5 atomes de carbone ou benzolye, que l'on cyclise pour obtenir un dérivé de formule (IV) :

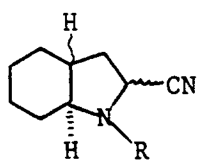

(IV)

dans laquelle R a la signification déjà indiquée que l'on hydrolyse sélectivement la fonction amide à l'aide d'une solution aqueuse diluée d'un acide minéral, pour obtenir un dérivé de formule (IV') :

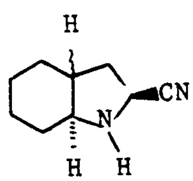

(IV')

puis hydrolyse le nitrile à l'aide d'un acide minéral en solution aqueuse concentrée pour obtenir le sel correspondant du dérivé de formule (I) recherché que l'on isole, si désiré, ou libère de son sel et isole si désiré ou salifie différemment et isole si désiré.

Les sels d'addition avec les acides peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques ou arylcarboxyliques.

Par procédé de préparation asymétrique, on entend que les configurations absolues des carbones 3a et 7a sont conservées dans le cours du procédé. C'est ainsi, par exemple qu'un dérivé de formule (II) 3a(R)-trans conduit à un dérivé de formule (I) 3a(R)trans. La présente demande vise aussi bien la préparation des produits cis que trans, et de préférence, ces derniers, notamment 3aR.

La réaction d'Hofmann conduisant au dérivé de formule (II) décarbonylé est réalisée dans les conditions classiques (cf. par exemple Merck Index 9ème Ed. p. ONR 45.

Le formaldéhyde est de préférence utilisé tel quel, mais peut être utilisé en combinaison bisulfitique, ou encore polymérisé, notamment sous forme de paraformaldéhyde.

Les ions cyanures sont de préférence apportés par un cyanure alcalin, tel que le cyanure de sodium ou de préférence de potassium.

L' halogénure d'acyle aliphatique ou de benzoyle est un iodure, un bromure, ou de préférence un

chlorure. L'acyle aliphatique renferme de 1 à 5 atomes de carbone, mais de préférence 2 ou 3.

On préfère utiliser un halogénure de benzoyle notamment le chlorure.

Toutes ces opérations sont de préférence utilisées dans le même récipient sans isoler les intermédiaires réactionnels. Ceux-ci peuvent l'être cependant, si désiré.

La cyclisation du dérivé de formule (III) est réalisée de préférence par réaction avec un halogénure d'alkyl sulfonyle ou d'alkylphényl sulfonyle suivie de l'action d'une base forte.

L'halogénure d'alkyl sulfonyle peut être un iodure, un bromure, et de préférence un chlorure. L'alkyle peut avoir de 1 à 3 atomes de carbone, et de préférence 1 seul.

On préfère le chlorure de méthane sulfonyl (ou de mésyle). L'halogénure d'alkylphénylsulfonyle peut être un iodure, un bromure, et de préférence un chlorure. L'alkyle peut avoir de 1 à 3 atomes de carbone, et de préférence 1 seul. On peut citer en particulier le chlorure de (4-méthylphényl) sulfonyle (ou tosyle).

La base forte est de préférence un hydrure alcalin tel que l'hydrure de potassium, et notamment de sodium. On peut utiliser également d'autres bases tel qu'un alcoolate alcalin, de préférence le tert-butylate de potassium.

L'hydrolyse en deux temps du dérivé de formule (IV) permet d'obtenir le bon isomère avec un rendement de 80 à 98% en partant d'un dérivé de formule (IV) dans lequel le bon isomère est en proportion de 0 à 70% seulement.

Dans le premier temps pour hydrolyser sélectivement l'amide, on utilise par exemple entre 1,5 et 3,0 équivalents de préférence environ 2,5 équivalents d'un acide minéral dilué tel que l'acide sulfurique, l'acide bromhydrique, et notamment l'acide chlorhydrique. On utilise par exemple l'acide sous une concentration 1 à 3N.

On peut opérer à température inférieure ou égale au reflux du mélange réactionnel, selon la concentration de l'acide.

Dans le second temps pour hydrolyser le nitrile, de préférence on ajoute environ 2 à 5 équivalents voire plus d'un acide, sous forme plus concentrée. On opère de préférence au reflux du mélange réactionnel sous une concentration de 3N ou plus, de préférence 5 ou 6N. L'acide peut être différent de celui utilisé dans le premier temps, mais est de préférence le même.

Dans ces conditions, en partant d'un mélange contenant 0% du bon isomère, on obtient environ 80% de bon isomère après hydrolyse, alors qu'on obtient 95% en partant d'un mélange renfermant 70% de bon isomère au départ.

Le produit de formule (I) obtenu sous forme salifiée peut être désalifié ou salifié différemment si désiré, selon les méthodes classiques.

Dans des conditions préférentielles de mise en oeuvre, le procédé ci-dessus décrit est caractérisé en ce que le dérivé de formule (II) est préparé soit par ouverture et épimérisation de la lactone de formule (V) :

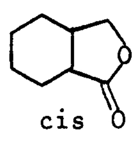

(V)

suivie d'une estérification pour obtenir un dérivé de formule (VI):

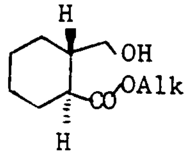

(VI)

dans laquelle Alk représente un radical alkyle renfermant de 1 à 5 atomes de carbone, que l'on amidifie pour obtenir le dérivé de formule (II) attendu de structure ($II_A$) :

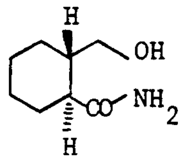

($II_A$)

soit par ouverture et amidification simultanées de ladite lactone de formule (V) pour obtenir le dérivé de formule (II) attendu de structure ($II_B$) :

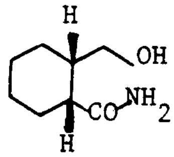

($II_B$)

L'ouverture et l'épimérisation de la lactone de formule (V) sont réalisées par exemple par action d'une amine secondaire linéaire ou ramifiée telle que la diéthylamine, et de préférence cyclisée telle que la morpholine, la pipéridine ou notamment la pyrrolidine.

On opère de préférence en présence d'une quantité catalytique d'un alcoolate alcalin renfermant de 1 à 5 atomes de carbone, n otamment le méthylate de sodium, dans un solvant tel que l'alcanol correspondant à l'alcoolate alcalin.

L'estérification est réalisée de préférence par mise en solution dans l'alkanol choisi pour l'estérification, comme l'isopropanol, le propanol et notamment l'éthanol et tout particulièrement le méthanol, en milieu acide minéral ou organique tel que l'acide sulfurique concentré ou l'acide paratoluène sulfonique.

L'amidification est faite à l'aide d'ammoniaque concentrée.

L'ouverture de la lactone et l'amidification simultanée sont réalisées à l'aide d'ammoniaque concentrée.

Le produit de formule (V) est connu. (Voir notamment P.D. Kennewell et Coll. Journal of Chemical Soc. Perkin I, 1982, 2563).

Dans d'autres conditions préférentielles, le produit de formule (V) est préparé à partir de l'anhydride de formule (VIII) :

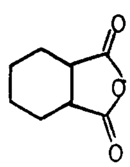

(VII)

- soit par action d'un excès d'un alkanol renfermant de 1 à 5 atomes de carbone, pour obtenir un dérivé de formule (VIII) :

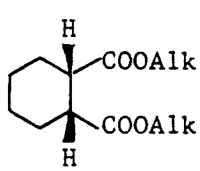

(VIII)

dans laquelle Alk a la signification déjà indiquée, que l'on soumet à l'action d'estérase de foie de porc pour obtenir un dérivé de formule (IX) :

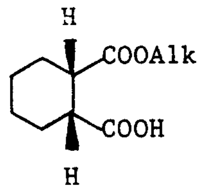

(IX)

dans laquelle Alk a la signification déjà indiquée, que l'on réduit par action de sodium dans l'ammoniaque ou de diéthyldihydroaluminate de sodium pour obtenir le dérivé de formule (V) cherché ;

- soit par action, en proportions sensiblement stoéchiométriques d'un alkanol renfermant de 1 à 5 atomes de carbone, pour obtenir un mélange de dérivés de formule (IX) que l'on traite comme indiqué ci-dessus pour obtenir le dérivé de formule (V) cherché, et de formule (X) :

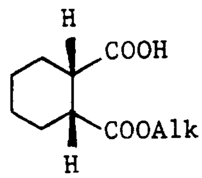

(X)

dans laquelle Alk a la signification déjà indiquée, que l'on réduit à l'aide d'un borohydrure alcalin après action d'un halogénoformiate d'alkyle $C_{1-3}$ pour obtenir le dérivé de formule (V) cherché.

La réaction de l'anhydride de formule (VII) avec l'alkanol en excès est réalisée de préférence en présence d'un acide minéral tel que l'acide chlorhydrique, et de préférence l'acide sulfurique. On fait réagir environ 2 moles d'alkanol pour 1 mole d'anhydride.

La réaction du dérivé de formule (VIII) avec l'estérase de foie de porc est réalisée par exemple dans les conditions décrites dans Angew. Chem. Int. Ed. Engl. 23 (1984), n¤ 1, p. 67-68.

Les dérivés de formule (IX) et (X) sont séparés notamment par dédoublement chimique et de préférence par recristallisation des sels diastéréoisomères des bases diméthyliques droite ou gauche du chloramphénicol.

La présente demande a également pour objet les produits de formule (III) :

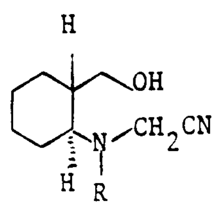

(III)

dans laquelle R a la signification déjà indiquée ainsi que de formule (VI) :

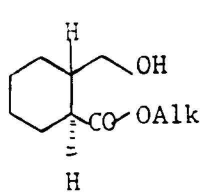

(VI)

dans laquelle Alk a la signification déjà indiquée.

Le procédé et les intermédiaires, objet de la présente demande, permettent notamment de préparer les inhibiteurs de l'enzyme de conversion de l'angiotensine de formule :

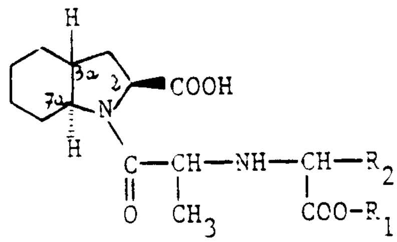

dans laquelle $R_1$ = $C_{1-6}$ alkyle, notamment $R_1$ = éthyle, et $R_2$ = $C_{1-6}$ alkyle ou phényle, $C_{1-4}$ alkyle, notamment $R_2$ = propyle ou $R_2$ = phényléthyle, et ou l'hydrogène en 3a est en position 3 alpha (dérivés cis) ou notamment 3 béta (dérivés trans).

Les exemples qui suivent illustrent l'invention sans toutefois la limiter.

**Exemple 1 : Chlorhydrate de L'acide [**(2 alpha, 3a alpha, 7a béta)**] octahydro 1H-indol-2-carboxylique.**
**Stade A :** N-(cyanométhyl) N-[(trans) 2-hydroxyméthylcyclohexyl] benzamide.
Dégradation d'Hofmann.

On ajoute en 10 minutes sous atmosphère inerte 3,6 cm3 de brome à 60 cm3 de soude 5N à 5¤C, agite la solution obtenue à 5¤C pendant 15 minutes, ajoute 10 g de (1S,trans) 2-(hydroxyméthyl) cyclohexane-carboxamide, agite 15 minutes, enlève le bain réfrigérant, agite encore 30 minutes, place dans un bain d'huile, chauffe pendant 30 minutes à 75¤C, refroidit à 10¤C et ajuste à pH 7 par introduction modérée de 17 cm3 d'acide chlorhydrique concentré 36¤Bé. On ajoute alors 4,4 cm3 de solution à 40% de formol dans l'eau et 4,15 g de cyanure de potassium, agite à température ambiante pendant 5 heures, puis 16 heures à 5¤C, ajoute ensuite à 20¤C, 8 g de carbonate de sodium, 50 cm3 d'acétate d'éthyle et 8,5 cm3 de chlorure de benzoyle, agite pendant 20 minutes, filtre, extrait à l'acétate d'éthyle, lave à l'eau, sèche, filtre, amène à sec sous pression réduite, purifie par chromatographie sur silice (éluant : cyclohexane-acétate d'éthyle 1-1) et obtient 7,76 g du produit attendu, F = 113¤C après recristallisation dans l'éther isopropylique.

**Stade B :** 1-benzoyl-octahydro-1H-indol-2-carbonitrile.

On prépare sous agitation et atmosphère inerte une solution de 7,2 g du produit du stade A dans 36 cm3 de tétrahydrofuranne, refroidit à 5¤C, ajoute 4,3 cm3 de triéthylamine puis 2,37 cm3 de chlorure de méthane sulfonyle, agite 1 heure à 5¤C, ajoute 14,4 cm3 de diméthylformamide et 5,25 g d'hydrure de sodium à 50%, agite pendant 16 heures à 20¤C, refroidit à 5¤C, ajoute peu à peu 10 cm3 de mélange à parties égales d'eau et de tétrahydrofuranne, dilue à l'acétate d'éthyle, verse sur 100 cm3 de phosphate monosodique, filtre, extrait à l'acétate d'éthyle, lave à l'eau, sèche, filtre, amène à sec sous pression réduite et obtient 9,4 g du produit attendu que l'on purifie par chromatographie sur silice (éluant : cyclohexane-acétate d'éthyle 1-1) et obtient 4,43 g du produit attendu. F = 125¤C après recristallisation dans l'éther isopropylique. Par recristallisation dans le chlorure de méthylène-éther isopropylique 2-5, on obtient le produit attendu comprenant 96% de l'épimère possèdant le radical CN en béta. PF du mélange 96% béta - 4% alpha ≈ 135¤C.

Variante du stade B.

Le stade B peut être réalisé en 2 étapes en isolant le dérivé intermédiaire.

On opère comme ci-dessus en utilisant comme solvant 10 volumes de chlorure de méthylène mais en agitant pendant 2 heures 30 minutes à 5¤C. On obtient le N-(cyanométhyl) N-[(trans) 2-méthyl sulfonyloxy-méthyl cyclohexyl] benzamide intermédiaire. F = 115¤C après cristallisation dans l'éther isopropylique. Après traitement à l'hydrure de sodium, dans le diméthylformamide, on obtient le produit attendu. F = 115¤C, CN en béta : 70%, CN en alpha : 30%.

**Stade C** : Chlorhydrate de l'acide [(2 alpha, 3a alpha, 7a béta)] octahydro 1H-indol-2-carboxylique.

On dissout sous agitation et atmosphère inerte 500 mg de produit du stade B (mélange CN béta-CN alpha 70-30) dans 1,5 cm3 de dioxanne, ajoute 0,95 cm3 d'acide chlorhydrique concentré 36¤ Bé dilué au

demi, agite 1 heure 30 minutes au reflux (≈92¤/C), introduit ensuite 0,42 cm3 d'acide chlorhydrique concentré, porte 1 heure au reflux, refroidit à température ambiante, dilue par 1 cm3 d'eau, élimine l'acide benzoïque par double extraction avec chaque fois 5 cm3 d'acétate d'éthyle, lave par 0,5 cm3 d'eau, évapore à sec les phases aqueuses, fait un entraînement au chlorure de méthylène, reprend au chlorure de méthylène, essore, lave, sèche à 20¤C sous pression réduite et obtient 465 mg du produit attendu. COOH béta = 96%. En opérant comme ci-dessus mais à partir d'un produit du stade B, CN alpha = 100%, on obtient le produit attendu, COOH béta = 83%.

**Préparation du (1S,trans) 2-(hydroxyméthyl) cyclohexane carboxamide.**

Stade 1 : (1S,trans) 1-[[2-(hydroxyméthyl) 1-cyclohexyl] carbonyl] pyrrolidine.

On porte au reflux pendant 20 heures, 4 g de (3aS,cis) hexahydro 1-(3H)-isobenzofuranone dans 20 cm3 de méthanol et 4,76 cm3 de pyrrolidine avec 0,386 g de méthylate de sodium, distille la moitié du méthanol à 50¤C sous pression réduite, ajoute de l'eau, de la glace, acidifie à pH = 1 par de l'acide chlorhydrique 2N, extrait au chlorure de méthylène, lave à l'eau, sèche, filtre, évapore à sec sous pression réduite, cristallise dans l'éther isopropylique et obtient 3,89 g du produit attendu. F = 115¤C.

Stade 2 : (1S,trans) 2-(hydroxyméthyl) cyclohexane carboxylate de méthyle.

On porte 8 heures au reflux 790 mg du produit du stade précédent dans 8 cm3 de méthanol, 0,2 cm3 d'acide sulfurique (d = 1,84), distille une partie du méthanol, verse sur de l'eau glacée, extrait au chlorure de méthylène, lave à l'eau, sèche, filtre, amène à sec sous pression réduite, purifie par chromatographie sur silice (éluant : chlorure de méthylèneacétate d'éthyle 9-1) et obtient 506 mg du produit attendu.
/alpha/$_D$ = +27,5¤ (c = 0,5%, chloroforme).

Stade 3 : (1S,trans) 2-(hydroxyméthyl) cyclohexane carboxamide.

On agite pendant 16 heures 200 mg du produit du stade précédent dans 2 cm3 d'ammoniaque concentrée, essore, dissout le précipité dans du chlorure de méthylène à 10% de méthanol, concentre à sec, reprend à l'éther isopropylique, concentre à faible volume, essore, lave avec très peu d'éther isopropylique et obtient 134 mg du produit attendu. F = 170¤C.
/alpha/$_D$ = +45¤ (c = 0,9%, méthanol).

**Préparation du (1S,cis) 2-(hydroxyméthyl) cyclohexane carboxamide.**

On agite pendant 24 heures à température ambiante 250 mg de (3aS,cis) hexahydro 1 -(3H)-isobenzofuranone dans 2,5 cm3 d' ammoniaque concentrée 28¤ Bé, essore le précipité formé, le sèche, et obtient 150 mg du produit attendu. F = 153¤C.

Ce produit cis, traité comme indiqué à l'exemple 1, conduit au dérivé (3a 7a) cis correspondant.

**Préparation de la (3aS,cis) hexahydro 1-(3H)-isobenzofuranone.**

Stade A : diméthyl ester de l'acide méso cis 1,2-cyclohexane dicarboxylique.

On porte pendant 20 heures au reflux 100 g d'anhydride hexahydrophtalique dans 78 cm3 d'orthoformiate de méthyle et 325 cm3 de méthanol en présence de 2,6 cm3 d'acide sulfurique concentré, pour obtenir 123,3 g du produit attendu.

Stade B : ester monométhylique de l'acide (1S,cis) 1,2-cyclohexane dicarboxylique.

On porte à 30¤C sous agitation 70 g de produit du stade A dans 175 cm3 d'eau et 175 cm3 de tampon phosphate pH = 7 (0,2M) avec 4 cm3 d'estérase de foie de porc (40 mg) (Boehringer Mannheim) pendant 48 heures en maintenant le pH à 7 par addition de soude N, ajoute 3 cm3 d'estérase, puis encore 3 cm3 au bout de 96 heures. Au bout de 120 heures, on extrait à l'acétate d'éthyle, acidifie à pH = 2 les eaux mères par de l'acide chlorhydrique concentré, extrait à l'acétate d'éthyle, sèche, filtre, amène à sec sous pression réduite et obtient 61 g du produit attendu. F<50¤C.

Stade A′ : Ester monométhylique de l'acide (dl,cis) 1,2-cyclohexane dicarboxylique.

On porte au reflux pendant 2 heures 30 minutes, 30 g d'anhydride hexahydrophtalique dans 300 cm3 de méthanol, évapore à sec sous pression réduite, chromatographie le résidu sur silice (éluant : cyclohexane-acétate d'éthyle 6-4) et recristallise dans l'hexane pour obtenir 32,6 g du produit attendu.

Stade B′1 : Ester monométhylique de l'acide (1S,cis) 1,2-cyclohexane dicarboxylique.

On ajoute 2,883 g de base droite diméthylique de chloramphénicol à une solution de 2,235 g de produit du stade A′ dans 40 cm3 de chlorure de méthylène, ajoute 40 cm3 d'acétate d'éthyle, distille sous pression réduite le chlorure de méthylène, obtient 2,655 g de produit cristallisé. On le dissout dans du chlorure de méthylène, ajoute de la glace, environ 80 cm3 d'acide chlorhydrique 0,1N, extrait au chlorure de méth ne, évapore à sec, cristallise dans l'hexane à -60¤C et obtient 0,87 g du produit attendu. F = 50¤C.
/alpha/ $_D$ = +4¤ (c = 1%, méthanol).

Stade B′2 : Ester monométhylique de l'acide (1R,cis) 1,2-cyclohexane dicarboxylique.

On opère comme ci-dessus, à partir de 2,402 g de base gauche diméthylénique du chloramphénicol et 1,862 g de produit du stade A′ et obtient 2,216 g de produit sous forme de sel de la base optique. A partir du sel obtenu, on obtient 0,736 g du produit attendu. F = 50¤C.
/alpha/ $_D$≃ -6¤ (c = 0,3% méthanol).

Stade C : (3aS,cis) hexahydro 1-(3H)-isobenzofuranone.
Variante 1 :

On condense 400 cm3 d'ammoniac à -65¤C, ajoute une solution de 14,6 g du produit du stade B ou B′1 dans 40 cm3 d'éthanol, ajoute par fractions sous agitation 18 g de sodium, agite encore 30 minutes après la fin de l'introduction, ajoute lentement 120 cm3 d'éthanol, laisse s'évaporer l'ammoniac pendant 16 heures et dissout le solide blanc obtenu, par addition de 60 cm3 d'éthanol, puis de l'acétate d'éthyle, puis de l'eau. On acidifie à l'aide de 68 cm3 d'acide chlorhydrique concentré, extrait à l'acétate d'éthyle, lave, sèche et concentre à sec. On porte au reflux 5,5 g du résidu ainsi obtenu dans 70 cm3 de toluène avec 165 mg d'acide paratoluène sulfonique, distille pour éliminer l'eau, refroidit, ajoute de la glace et du bicarbonate de sodium, extrait à l'acétate d'éthyle, lave, sèche, filtre, amène à sec, purifie par chromatographie sur silice (éluant : cyclohexane-acétate d'éthyle) et obtient 2,9 g du produit attendu. Eb = 70¤C sous 1 mm de mercure.
/Alpha/ $_D$ = -26,5¤ (c = 1,5%, méthanol).

Variante 2 :

On ajoute à -5¤C en quelques minutes une solution de 256 mg de chloroformiate d'éthyle dans 0,6 cm3 de tétrahydrofuranne, à une solution de 440 mg de produit du stade B′2 et de 239 mg de triéthylamine dans 3,3 cm3 de tétrahydrofuranne, agite pendant 30 minutes puis élimine le précipité. On ajoute goutte à goutte le filtrat à une suspension de 235 mg de borohydrure de sodium à 95% dans 2,3 cm3 d'eau maintenue à environ 12¤C, laisse ensuite 3 heures 30 minutes à température ambiante, glace, acidifie à l'aide d'acide chlorhydrique 2N, extrait au chlorure de méthylène, sèche, amène à sec, purifie par chromatographie sur silice (éluant : cyclohexane-acétate d'éthyle 65-35) et obtient 228 mg du produit attendu.
/Alpha/ $_D$ = -47¤C (c = 0,6%, chloroforme).

**Revendications**
**Revendications pour les Etats Contractants suivants : AT, BE, CH, DE, GB, GR, IT, LI, LU, NL, SE**

**1.** Procédé de préparation asymétrique des dérivés de formule (I) :

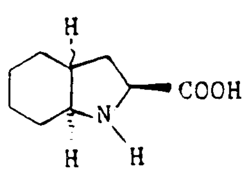

(I)

dans laquelle les hydrogènes en 3a et 7a sont de configuration cis ou trans, ainsi que de leurs sels d'addition avec les acides, caractérisé en en que l'on soumet un produit de formule (II) :

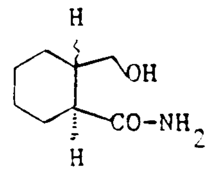

(II)

à une réaction d'Hofmann, puis à l'action de formaldéhyde en présence d'ions cyanures et d'un halogénure d'acyle aliphatique renfermant de 1 à 5 atomes de carbone ou de benzoyle, pour obtenir un dérivé de formule (III) :

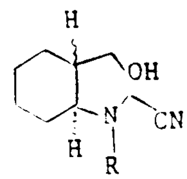

(III)

dans laquelle R représente un radical acyle aliphatique renfermant de 1 à 5 atomes de carbone ou benzoyle, que l'on cyclise par action d'un halogénure d'alkyl sulfonyle ou d'alkylphényl sulfonyle suivie de l'action d'une base forte pour obtenir un dérivé de formule (IV) :

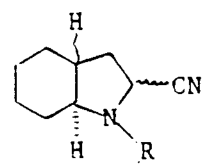

(IV)

dans laquelle R a la signification déjà indiquée que l'on hydrolyse sélectivement la fonction amide à l'aide d'une solution aqueuse diluée d'un acide minéral, pour obtenir un dérivé de formule (IV'):

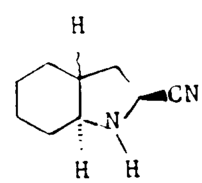

(IV')

puis hydrolyse le nitrile à l'aide d'un acide minéral en solution aqueuse concentrée pour obtenir le sel correspondant du dérivé de formule (I) recherché que l'on isole, si désiré, ou libère de son sel et isole si désiré ou salifie différemment et isole si désiré.

2. Procédé selon la revendication 1, caractérisé en ce que les hydrogènes en 3a et 7a sont de configuration trans.

3. Procédé selon la revendication 1 ou 2, caractérisé en ce que le dérivé de formule (II) est préparé soit par ouverture et épimérisation de la lactone de formule (V) :

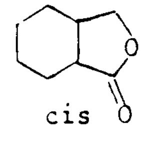

(V)

suivie d'une estérification pour obtenir un dérivé de formule (VI):

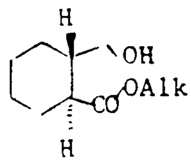

(VI)

dans laquelle Alk représente un radical alkyle renfermant de 1 à 5 atomes de carbone, que l'on amidifie pour obtenir le dérivé de formule (II) attendu de structure ($II_A$) :

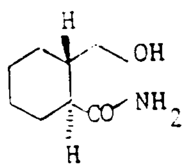

($II_A$)

soit par ouverture et amidification simultanées de ladite lactone de formule (V) pour obtenir le dérivé de formule (II) attendu de structure ($II_B$) :

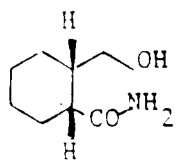

($II_B$)

**4.** Procédé selon la revendication 3, caractérisé en ce que :
- l'ouverture et l'épimérisation sont réalisées par action d'une amine secondaire en présence d'un alcoolate alcalin renfermant de 1 à 5 atomes de carbone ;
- l'estérification est réalisée en milieu acide dans l'alcanol renfermant de 1 à 5 atomes de carbone choisi pour l'estérification ;
- l'amidification est réalisée à l'aide d'ammoniaque concentrée.
- l'ouverture de la lactone et l'amidification simultanée sont realisées à l'aide d'ammoniaque concentrée.

**5.** Les produits de formule (III) :

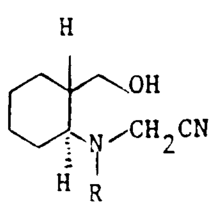

(III)

dans laquelle R a la signification déjà indiquée.

**6.** Les produits de formule (VI) :

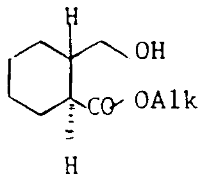

(VI)

dans laquelle Alk a la signfication déjà indiquée.

**7.** Procédé selon la revendication 3 caractérisé en ce que la lactone de formule (V) est préparée à partir de l'anhydride de formule (VII) :

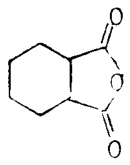

(VII)

- soit par action d'un excès d'un alkanol renfermant de 1 à 5 atomes de carbone, pour obtenir un dérivé de formule (VIII) :

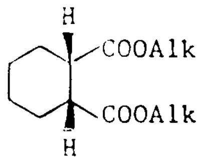

(VIII)

dans laquelle Alk a la signification déjà indiquée, que l'on soumet à l'action d'estérase de foie de porc pour obtenir un dérivé de formule (IX) :

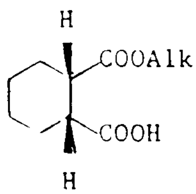

(IX)

dans laquelle Alk a la signification déjà indiquée, que l'on réduit par action de sodium dans l'ammoniaque ou de diéthyldihydroaluminate de sodium pour obtenir le dérivé de formule (V) cherché ;

- soit par action, en proportions sensiblement stoéchiométriques d'un alkanol renfermant de 1 à 5 atomes de carbone, pour obtenir un mélange de dérivés de formule (IX) que l'on traite comme indiqué ci-dessus pour obtenir le dérivé de formule (V) cherché, et de formule (X) :

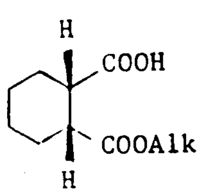

(X)

dans laquelle Alk a la signification déjà indiquée, que l'on réduit à l'aide d'un borohydrure alcalin après action d'un halogénoformiate d'alkyle $C_{1-3}$ pour obtenir le dérivé de formule (V) cherché.

**8.** Procédé selon la revendication 7, caractérisé en ce que l'estérase de foie de porc est sous la forme d'un broyat de foie de porc.

**Revendications pour l'Etat contractant suivant : ES**

**1.** Procédé de préparation asymétrique des dérivés de formule (I) :

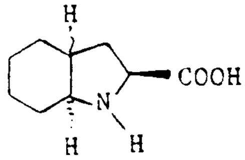

(I)

dans laquelle les hydrogènes en 3a et 7a sont de configuration cis ou trans, ainsi que de leurs sels d'addition avec les acides, caractérisé en en que l'on soumet un produit de formule (II) :

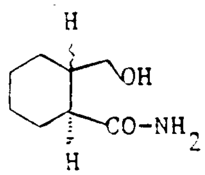

(II)

à une réaction d'Hofmann, puis à l'action de formaldéhyde en présence d'ions cyanures et d'un halogénure d'acyle aliphatique renfermant de 1 à 5 atomes de carbone ou de benzoyle, pour obtenir un dérivé de formule (III) :

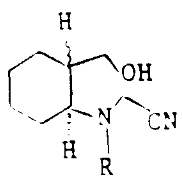

(III)

dans laquelle R représente un radical acyle aliphatique renfermant de 1 à 5 atomes de carbone ou benzoyle, que l'on cyclise par action d'un halogénure d'alkyl sulfonyle ou d'alkylphényl sulfonyle suivie de l'action d'une base forte pour obtenir un dérivé de formule (IV) :

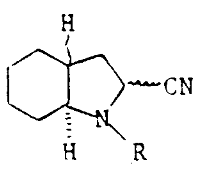

(IV)

dans laquelle R a la signification déjà indiquée que l'on hydrolyse sélectivement la fonction amide à l'aide d'une solution aqueuse diluée d'un acide minéral, pour obtenir un dérivé de formule (IV') :

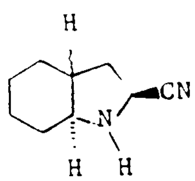

(IV')

puis hydrolyse le nitrile à l'aide d'un acide minéral en solution aqueuse concentrée pour obtenir le sel correspondant du dérivé de formule (I) recherché que l'on isole, si désiré, ou libère de son sel et isole si désiré ou salifie différemment et isole si désiré.

2. Procédé selon la revendication 1, caractérisé en ce que les hydrogènes en 3a et 7a sont de configuration trans.

3. Procédé selon la revendication 1 ou 2, caractérisé en ce que le dérivé de formule (II) est préparé soit par ouverture et épimérisation de la lactone de formule (V) :

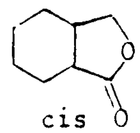

(V)

suivie d'une estérification pour obtenir un dérivé de formule (VI) :

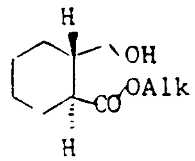

(VI)

dans laquelle Alk représente un radical alkyle renfermant de 1 à 5 atomes de carbone, que l'on amidifie pour obtenir le dérivé de formule (II) attendu de structure ($II_A$):

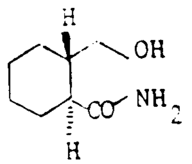

($II_A$)

soit par ouverture et amidification simultanées de ladite lactone de formule (V) pour obtenir le dérivé de formule (II) attendu de structure ($II_B$) :

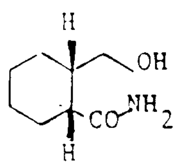

($II_B$)

4. Procédé selon la revendication 3, caractérisé en ce que :
   - l'ouverture et l'épimérisation sont réalisées par action d'une amine secondaire en présence d'un alcoolate alcalin renfermant de 1 à 5 atomes de carbone ;
   - l'estérification est réalisée en milieu acide dans l'alcanol renfermant de 1 à 5 atomes de carbone choisi pour l'estérification ;
   - l'amidification est réalisée à l'aide d'ammoniaque concentrée.
   - l'ouverture de la lactone et l'amidification simultanée sont réalisées à l'aide d'ammoniaque concentrée.

5. Procédé selon la revendication 3, caractérisé en ce que la lactone de formule (V) est préparée à partir de l'anhydride de formule (VII) :

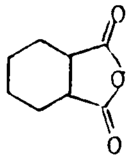

(VII)

- soit par action d'un excès d'un alkanol renfermant de 1 à 5 atomes de carbone, pour obtenir un dérivé de formule (VIII) :

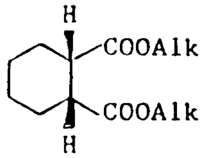

(VIII)

dans laquelle Alk a la signification déjà indiquée, que l'on soumet à l'action d'estérase de foie de porc pour obtenir un dérivé de formule (IX) :

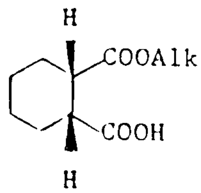

(IX)

dans laquelle Alk a la signification déjà indiquée, que l'on réduit par action de sodium dans l'ammoniaque ou de diéthyldihydroaluminate de sodium pour obtenir le dérivé de formule (V) cherché ;

- soit par action, en proportions sensiblement stoéchiométriques d'un alkanol renfermant de 1 à 5 atomes de carbone, pour obtenir un mélange de dérivés de formule (IX) que l'on traite comme indiqué ci-dessus pour obtenir le dérivé de formule (V) cherché, et de formule (X) :

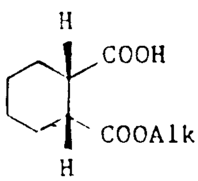

(X)

dans laquelle Alk a la signification déjà indiquée, que l'on réduit à l'aide d'un borohydrure alcalin après action d'un halogénoformiate d'alkyle $C_{1-3}$ pour obtenir le dérivé de formule (V) cherché.

**6.** Procédé selon la revendication 5, caractérisé en ce que l'estérase de foie de porc est sous la forme d'un broyat de foie de porc.

**Claims**
**Claims for the following Contracting States : AT, BE, CH, DE, GB, GR, IT, LI, LU, NL, SE**

**1.** Asymmetric preparation process for the derivatives of formula (I):

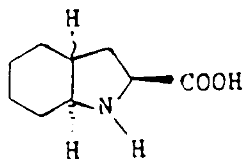

(I)

in which the hydrogens in positions 3a and 7a are of cis or trans configuration, as well as their addition salts with acids, characterized in that a product of formula (II):

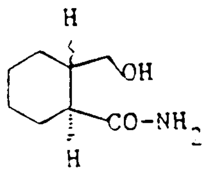

(II)

is subjected to a Hofmann reaction, then to the action of formaldehyde in the presence of cyanide ions and an aliphatic acyl halide containing 1 to 5 carbon atoms or a benzoyl halide, in order to obtain a derivative of formula (III):

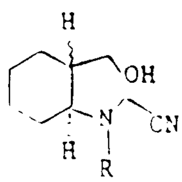

(III)

in which R represents an aliphatic acyl radical containing 1 to 5 carbon atoms or a benzoyl radical, which is cyclized by the action of an alkyl sulphonyl or alkylphenyl sulphonyl halide follows by the action of a strong base in order to obtain a derivative of formula (IV):

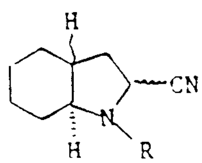

(IV)

in which R has the meaning already indicated, the amide function of which is selectively hydrolyzed using a dilute aqueous solution of a mineral acid, in order to obtain a derivative of formula (IV'):

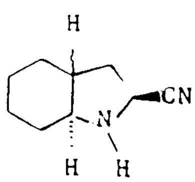

(IV')

then the nitrile is hydrolyzed using a mineral acid in a concentrated aqueous solution in order to obtain the corresponding salt of the desired derivative of formula (I) which is isolated, if desired, or freed from its salt and isolated if desired or salified in a different way and isolated if desired.

2. Process according to claim 1, characterized in that the hydrogens in positions 3a and 7a are of trans

configuration.

**3.** Process according to claim 1 or 2, characterized in that the derivative of formula (II) is prepared either by opening and epimerization of the lactone of formula (V):

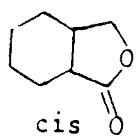

(V)

followed by an esterification in order to obtain a derivative of formula (VI):

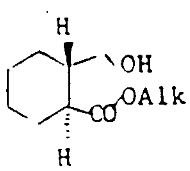

(VI)

in which Alk represents an alkyl radical containing 1 to 5 carbon atoms, which is amidified in order to obtain the expected derivative of formula (II) of structure ($II_A$):

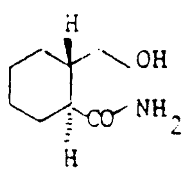

($II_A$)

or by simultaneous opening and amidification of said lactone of formula (V) in order to obtain the expected derivative of formula (II) of structure ($II_B$):

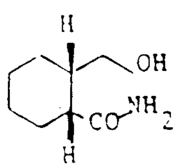

($II_B$)

**4.** Process according to claim 3, characterized in that:
- the opening and epimerization are carried out by the action of a secondary amine in the presence of an alkaline alcoholate containing 1 to 5 carbon atoms;
- the esterification is carried out in an acid medium in the alkanol containing 1 to 5 carbon atoms chosen for the esterification;
- the amidification is carried out using concentrated ammonium hydroxide;
- the opening of the lactone and the simultaneous amidification are carried out using concentrated ammonium hydroxide.

**5.** The products of formula (III):

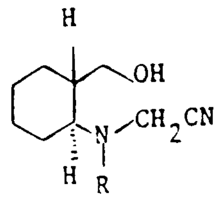

(III)

in which R has the meaning already indicated.

**6.** The products of formula (VI):

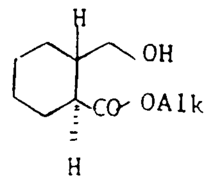

(VI)

in which Alk has the meaning already indicated.

**7.** Process according to claim 3, characterized in that the lactone of formula (V) is prepared from the anhydride of formula (VII);

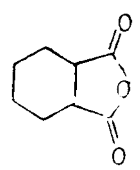

(VII)

- either by the action of an excess of an alkanol containing 1 to 5 carbon atoms, in order to obtain a derivative of formula (VIII):

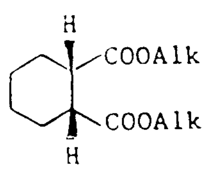

(VIII)

in which Alk has the meaning already indicated, which is subjected to the action of pork liver esterase in order to obtain a derivative of formula (IX):

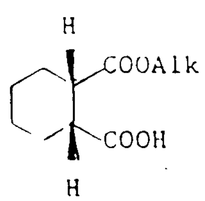

(IX)

in which Alk has the meaning already indicated, which is reduced by the action of sodium in

ammonium hydroxide or sodium diethyldihydroaluminate in order to obtain the desired derivative of formula (V);

- or by the action, in approximately stoichiometric proportions, of an alkanol containing 1 to 5 carbon atoms, in order to obtain a mixture of derivatives of formula (IX) which is treated as indicated above in order to obtain the desired derivative of formula (V) and of formula (X):

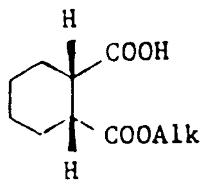

(X)

in which Alk has the meaning already indicated, which is reduced using an alkaline borohydride after the action of a $C_{1}$-$_{3}$ alkyl halogenoformate in order to obtain the desired derivative of formula (V).

**8.** Process according to claim 7, characterized in that the pork liver esterase is in the form of ground-up pork liver.

**Claims for the following Contracting State : ES**

**1.** Asymmetric preparation process for the derivatives of formula (I):

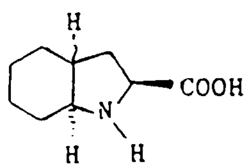

(I)

in which the hydrogens in positions 3a and 7a are of cis or trans configuration, as well as their addition salts with acids, characterized in that a product of formula (II):

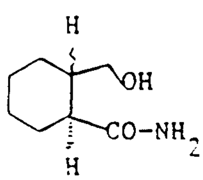

(II)

is subjected to a Hofmann reaction, then to the action of formaldehyde in the presence of cyanide ions and an aliphatic acyl halide containing 1 to 5 carbon atoms or a benzoyl halide, in order to obtain a derivative of formula (III):

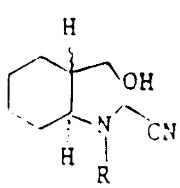

(III)

in which R represents an aliphatic acyl radical containing 1 to 5 carbon atoms or a benzoyl radical, which is cyclized by the action of an alkyl sulphonyl or alkylphenyl sulphonyl halide followed by the action of a strong base in order to obtain a derivative of formula (IV):

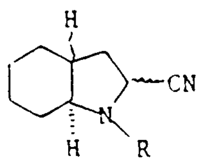

(IV)

in which R has the meaning already indicated, the amide function of which is selectively hydrolyzed using a dilute aqueous solution of a mineral acid, in order to obtain a derivative of formula (IV')

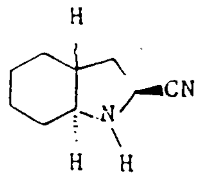

(IV')

the the nitrile is hydrolyzed using a mineral acid in a concentrated aqueous solution in order to obtain the corresponding salt of the desired derivative of formula (I) which is isolated, if desired, or freed from its salt and isolated if desired or salified in a different way and isolated if desired.

**2.** Process according to claim 1, characterized in that the hydrogens in positions 3a and 7a are of trans configuration.

**3.** Process according to claim 1 or 2, characterized in that the derivative of formula (II) is prepared either by opening and epimerization of the lactone of formula (V):

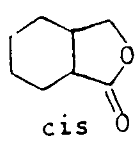

(V)

followed by an esterification in order to obtain a derivative of formula (VI):

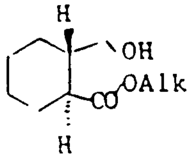

(VI)

in which Alk represents an alkyl radical containing 1 to 5 carbon atoms, which is amidified in order to obtain the expected derivative of formula (II) of structure ($II_A$):

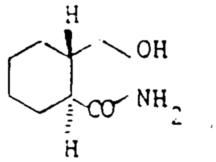

$(II_A)$

or by simultaneous opening and amidification of said lactone of formula (V) in order to obtain the expected derivative of formula (II) of structure ($II_B$):

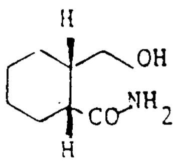

$(II_B)$

4. Process according to claim 3, characterized in that:
   - the opening and epimerization are carried out by the action of a secondary amine in the presence of an alkaline alcoholate containing 1 to 5 carbon atoms;
   - the esterification is carried out in an acid medium in the alkanol containing 1 to 5 carbon atoms chosen for the esterification;
   - the amidification is carried out using concentrated ammonium hydroxide;
   - the opening of the lactone and the simultaneous amidification are carried out using concentrated ammonium hydroxide.

5. Process according to claim 3, characterized in that the lactone of formula (V) is prepared from the anhydride of formula (VII):

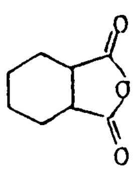

(VII)

- either by the action of an excess of an alkanol containing 1 to 5 carbon atoms, in order to obtain a derivative of formula (VIII):

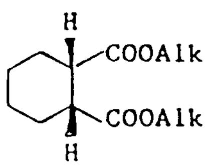

(VIII)

in which Alk has the meaning already indicated, which is subjected to the action of pork liver esterase in order to obtain a derivative of formula (IX):

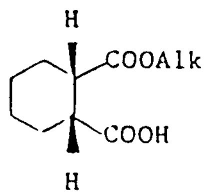

(IX)

in which Alk has the meaning already indicated, which is reduced by the action of sodium in ammonium hydroxide or sodium diethyldihydroaluminate in order to obtain the desired derivative of formula (V);

- or by the action, in approximately stoichiometric proportions, of an alkanol containing 1 to 5 carbon atoms, in order to obtain a mixture of derivatives of formula (IX) which is treated as indicated above in order to obtain the desired derivative of formula (V), and of formula (X):

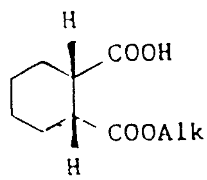

(X)

in which Alk has the meaning already indicated, which is reduced using an alkaline borohydride after the action of a $C_{1-3}$ alkyl halogenoformate in order to obtain the desired derivative of formula (V).

**6.** Process according to claim 5, characterized in that the pork liver esterase is in the form of ground-up pork liver.

**Patentansprüche**
**Patentansprüche für folgende Vertragsstaaten : AT, BE, CH, DE, GB, GR, IT, LI, LU, NL, SE**

**1.** Verfahren zur asymmetrischen Herstellung der Derivate der Formel (I)

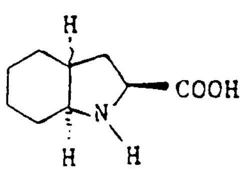

(I)

worin die Wasserstoffe in 3a- und 7a-Stellung die cis-oder trans-Konfiguration aufweisen, sowie von deren Additionssalzen mit Säuren, dadurch gekennzeichnet, daß man ein Produkt der Formel (II)

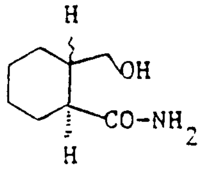

(II)

einer Hofmann-Reaktion, danach der Einwirkung von Formaldehyd in Gegenwart von Cyanidionen und eines aliphatischen Acylhalogenids mit 1 bis 5 Kohlenstoffatomen oder Benzoylhalogenid unterzieht, um zu einem Derivat der Formel (III)

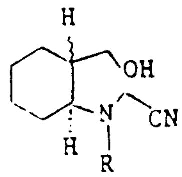

(III)

worin R für einen aliphatischen Acylrest mit 1 bis 5 Kohlenstoffatomen oder einen Benzoylrest steht, zu gelangen, welches man durch Einwirkung eines Alkylsulfonyl- oder Alkylphenylsulfonylhalogenids und anschließende Einwirkung einer starken Base cyclisiert, um zu einem Derivat der Formel (IV)

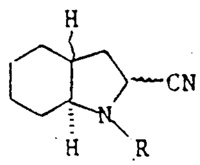

(IV)

worin R die vorstehend angegebene Bedeutung besitzt, zu gelangen, man selektiv die Amidfunktion mit einer verdünnten, wäßrigen Lösung eher Mineralsäure hydrolysiert, um zu einem Derivat der Formel (IV')

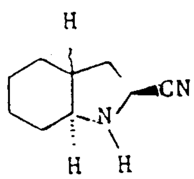

(IV')

zu gelangen, wonach man das Nitril mit einer Mineralsäure in konzentrierter, wäßriger Lösung hydrolysiert, um das entsprechende Salz des gewünschten Derivats der Formel (I) zu erhalten, welches man isoliert, wenn erwünscht, oder aus seinem Salz freisetzt und isoliert, wenn erwünscht, oder in ein anderes Salz überführt und isoliert, wenn erwünscht.

**2.** Verfahren gemäß Anspruch 1, dadurch gekennzeichnet, daß die Wasserstoffatome in 3a- und 7a-Stellung die trans-Konfiguration besitzen.

**3.** Verfahren gemäß Anspruch 1 oder 2, dadurch gekennzeichnet, daß das Derivat der Formel (II) entweder durch Öffnung und Epimerisierung des Lactons der Formel (V)

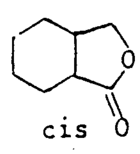

(V)

und anschließende Veresterung hergestellt wird, um ein Derivat der Formel (VI)

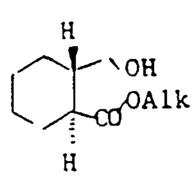

(VI)

worin Alk für einen Alkylrest mit 1 bis 5 Kohlenstoffatomen steht, zu erhalten, welches man amidifiziert, um zu dem erwarteten Derivat der Formel (II) der Struktur ($II_A$)

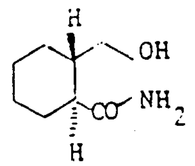

($II_A$)

zu gelangen, oder durch gleichzeitige Öffnung und Amidifizierung des Lactons der Formel (V) hergestellt wird, um zu dem erwarteten Derivat der Formel (II) mit der Struktur ($II_B$)

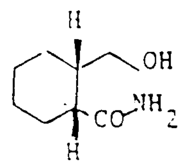

($II_B$)

zu gelangen.

**4.** Verfahren gemäß Anspruch 3, dadurch gekennzeichnet, daß
die Öffnung und die Epimerisierung durch Einwirkung eines sekundären Amins in Anwesenheit eines Alkalialkoholats mit 1 bis 5 Kohlenstoffatomen erfolgen;
die Veresterung in saurem Milieu in dem für die Veresterung ausgewählten Alkanol mit 1 bis 5 Kohlenstoffatomen durchgeführt wird;
die Amidifizierung mit Hilfe von konzentriertem Ammoniak erfolgt;
die Öffnung des Lactons und die gleichzeitige Amidifizierung mit Hilfe von konzentriertem Ammoniak erfolgen.

**5.** Die Produkte der Formel (III)

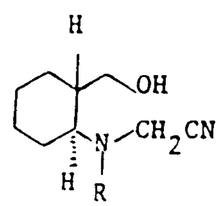

(III)

worin R die vorstehend angegebene Bedeutung besitzt.

**6.** Die Produkte der Formel (VI)

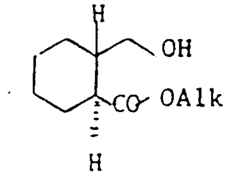

(VI)

worin Alk die vorstehend angegebene Bedeutung besitzt.

**7.** Verfahren gemäß Anspruch 3, dadurch gekennzeichnet, daß das Lacton der Formel (V) hergestellt wird

aus dem Anhydrid der Formel (VII)

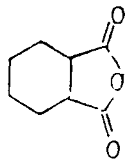

(VII)

entweder durch Einwirken eines Überschusses eines Alkanols mit 1 bis 5 Kohlenstoffatomen, um zu einem Derivat der Formel (VIII)

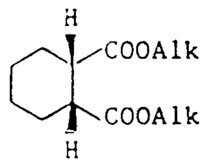

(VIII)

zu gelangen, worin Alk die vorstehend angegebene Bedeutung besitzt, das man der Einwirkung einer Schweineleberesterase unterzieht, um zu einem Derivat der Formel (IX)

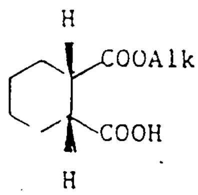

(IX)

zu gelangen, worin Alk die vorstehend angegebene Bedeutung besitzt, welches man durch Einwirkung von Natrium in Ammoniak oder Natriumdiethyldihydroaluminat reduziert, um zu dem gewünschten Derivat der Formel (V) zu gelangen;

oder durch Einwirken in im wesentlichen stöchiometrischen Anteilen eines Alkanols mit 1 bis 5 Kohlenstoffatomen, um zu einem Gemisch der Derivate der Formel (IX), das man, wie vorstehend angegeben, behandelt, um das gewünschte Derivat der Formel (V) zu erhalten, und der Formel (X)

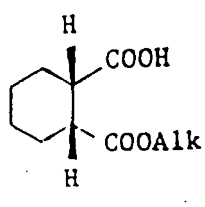

(X)

worin Alk die vorstehend angegebene Bedeutung besitzt, zu gelangen, welches man mit einem Alkaliborhydrid nach Einwirken eines $C_{1-3}$-Alkylhalogenoformiats reduziert, um zu dem gewünschten Derivat der Formel (V) zu gelangen.

**8.** Verfahren gemäß Anspruch 7, dadurch gekennzeichnet, daß die Schweineleberesterase in Form von vermahlener Schweineleber vorliegt.

**Patentansprüche für folgenden Vertragsstaat : ES**

**1.** Verfahren zur asymmetrischen Herstellung der Derivate der Formel (I)

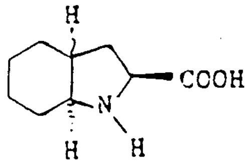

(I)

worin die Wasserstoffe in 3a- und 7a-Stellung die cis-oder trans-Konfiguration aufweisen, sowie von deren Additionssalzen mit Säuren, dadurch gekennzeichnet, daß man ein Produkt der Formel (II)

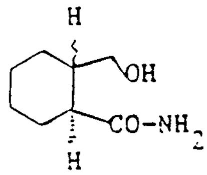

(II)

einer Hofmann-Reaktion, danach der Einwirkung von Formaldehyd in Gegenwart von Cyanidionen und eines aliphatischen Acylhalogenids mit 1 bis 5 Kohlenstoffatomen oder Benzoylhalogenid unterzieht, um zu einem Derivat der Formel (III)

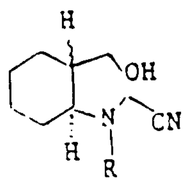

(III)

worin R für einen aliphatischen Acylrest mit 1 bis 5 Kohlenstoffatomen oder einen Benzoylrest steht, zu gelangen, welches man durch Einwirkung eines Alkylsulfonyl- oder Alkylphenylsulfonylhalogenids und anschließende Einwirkung einer starken Base cyclisiert, um zu einem Derivat der Formel (IV)

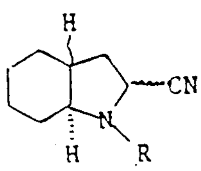

(IV)

worin R die vorstehend angegebene Bedeutung besitzt, zu gelangen, man selektiv die Amidfunktion mit einer verdünnten, wäßrigen Lösung eher Mineralsäure hydrolysiert, um zu einem Derivat der Formel (IV')

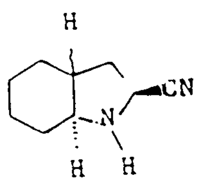

(IV')

zu gelangen, wonach man das Nitril mit einer Mineralsäure in konzentrierter, wäßriger Lösung hydrolysiert, um das entsprechende Salz des gewünschten Derivats der Formel (I) zu erhalten, welches man isoliert, wenn erwünscht, oder aus seinem Salz freisetzt und isoliert, wenn erwünscht, oder in ein anderes Salz überführt und isoliert, wenn erwünscht.

2. Verfahren gemäß Anspruch 1, dadurch gekennzeichnet, daß die Wasserstoffatome in 3a- und 7a-Stellung die trans-Konfiguration besitzen.

3. Verfahren gemäß Anspruch 1 oder 2, dadurch gekennzeichnet, daß das Derivat der Formel (II) entweder durch Öffnung und Epimerisierung des Lactons der Formel (V)

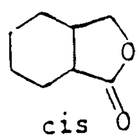

(V)

und anschließende Veresterung hergestellt wird, um ein Derivat der Formel (VI)

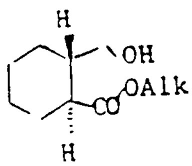

(VI)

worin Alk für einen Alkylrest mit 1 bis 5 Kohlenstoffatomen steht, zu erhalten, welches man amidifiziert, um zu dem erwarteten Derivat der Formel (II) der Struktur ($II_A$)

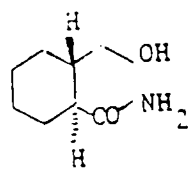

($II_A$)

zu gelangen, oder durch gleichzeitige Öffnung und Amidifizierung des Lactons der Formel (V) hergestellt wird, um zu dem erwarteten Derivat der Formel (II) mit der Struktur ($II_B$)

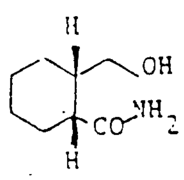

($II_B$)

zu gelangen.

4. Verfahren gemäß Anspruch 3, dadurch gekennzeichnet, daß

die Öffnung und die Epimerisierung durch Einwirkung eines sekundären Amins in Anwesenheit eines Alkalialkoholats mit 1 bis 5 Kohlenstoffatomen erfolgen;

die Veresterung in saurem Milieu in dem für die Veresterung ausgewählten Alkanol mit 1 bis 5 Kohlenstoffatomen durchgeführt wird;

die Amidifizierung mit Hilfe von konzentriertem Ammoniak erfolgt;

die Öffnung des Lactons und die gleichzeitige Amidifizierung mit Hilfe von konzentriertem Ammoniak erfolgen.

5. Verfahren gemäß Anspruch 3, dadurch gekennzeichnet, daß das Lacton der Formel (V) hergestellt wird aus dem Anhydrid der Formel (VIII)

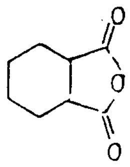

(VII)

entweder durch Einwirken eines Überschusses eines Alkanols mit 1 bis 5 Kohlenstoffatomen, um zu einem Derivat der Formel (VIII)

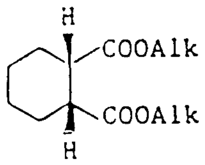

(VIII)

zu gelangen, worin Alk die vorstehend angegebene Bedeutung besitzt, das man der Einwirkung einer Schweineleberesterase unterzieht, um zu einem Derivat der Formel

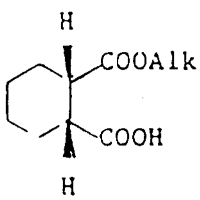

(IX)

zu gelangen, worin Alk die vorstehend angegebene Bedeutung besitzt, welches man durch Einwirkung von Natrium in Ammoniak oder Natriumdiethyldihydroaluminat reduziert, um zu dem gewünschten Derivat der Formel (V) zu gelangen;

oder durch Einwirken in im wesentlichen stöchiometrischen Anteilen eines Alkanols mit 1 bis 5 Kohlenstoffatomen, um zu einem Gemisch der Derivate der Formel (IX), das man, wie vorstehend angegeben, behandelt, um das gewünschte Derivat der Formel (V) zu erhalten, und der Formel (X)

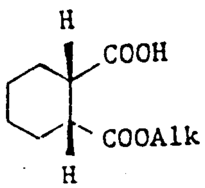

(X)

worin Alk die vorstehend angegebene Bedeutung besitzt, zu gelangen, welches man mit einem Alkaliborhydrid nach Einwirken eines $C_{1-3}$-Alkylhalogenoformiats reduzierte, um zu dem gewünschten Derivat der Formel (V) zu gelangen.

6. Verfahren gemäß Anspruch 5, dadurch gekennzeichnet, daß die Schweineleberesterase in Form von vermahlener Schweineleber vorliegt.